# EUROPEAN PATENT APPLICATION

(11) **EP 1 048 660 A1**
(43) Date of publication of application: **02.11.2000**
(21) Application number: 00109029.9
(22) Date of filing: 27.04.2000
(51) Int. Cl.: C07D 301/10, C07D 303/04

(54) **Manufacturing method of epoxides**

(30) Priority: 28.04.1999 JP 12121299
(71) Applicant: Agency of Industrial Science and Technology, Tokyo 100-8912 (JP); Nippon Shokubai Co., Ltd., Osaka-shi, Osaka 541-0043 (JP)
(72) Inventor: Hayashi, Toshio, Kobe-shi, Hyogo 651-2242 (JP); Wada, Masahiro, Nishinomiya-shi, Hyogo 662-0061 (JP); Haruta, Masatake, Ikeda-shi, Osaka 563-0024 (JP); Tsubota, Susumu, Ashiya-shi, Hyogo 659-0016 (JP)
(74) Representative: Beier, Ralph

(57) **Abstract**

In order to provide a suitable method of manufacturing epoxides by partially unsaturated hydrocarbons, unsaturated hydrocarbons are partially oxidized by using a catalyst made by fixing fine gold particles onto titanium-containing oxides in the presence of molecular hydrogen, oxygen, and alcohols and/or ethers as a co-existing compound. The co-existing compound is present in a volume ratio of 1 × 10⁻⁴-50 to 1 with respect to molecular hydrogen. Lower alcohols having 6 or less carbon atoms are preferable as alcohols. Titanium-containing oxides forming the catalyst may further include at least one element selected from the group consisting of alkali metal, alkali earth metal, lanthanoid, and thallium.

## Description

### FIELD OF THE INVENTION

The present invention relates to a manufacturing method of epoxides by partially oxidizing unsaturated hydrocarbons in the presence of molecular hydrogen and oxygen by using a catalyst made by depositing fine gold particles on titanium-containing oxides.

### BACKGROUND OF THE INVENTION

A manufacturing method of epoxides by partially oxidizing unsaturated hydrocarbons in the presence of molecular hydrogen and oxygen by using a catalyst made by depositing fine gold particles on titanium-containing oxides has been known (Japanese Laid-open Patent Application No. 127550/1996 (Japanese Official Gazette, *Tokukaihei No. 8-127550*, publishing date: May 21, 1996), Japanese Laid-open Patent Application No. 5590/1998 (Japanese Official Gazette, *Tokukaihei No. 10-5590,* publishing date: January 13, 1998), Japanese Laid-open Patent Application No. 244156/1998 (Japanese Official Gazette, *Tokukaihei No. 10-244156,* publishing date: September 14, 1998), etc.).

However, according to the foregoing manufacturing method of epoxides, the catalyst shows an activity to some extent in the initial stage of the epoxidation reaction, but the catalyst becomes less active as the reaction proceeds with time. Thus, the above catalyst has a problem that its activity becomes unsatisfactory by the time the reaction reaches the steady state.

In addition, 1 mol of molecular hydrogen (hereinafter, referred to simply as hydrogen in some cases) is theoretically consumed in producing 1 mol of epoxides. However, actual consumption is two to ten times as much. The consumption increases markedly with increasing reaction temperatures. The reason why the consumption of hydrogen increases is not clear, but heterogeneity of particle sizes of the fine gold particles deposited onto the titanium-containing oxides is presumed as one of the reasons.

### SUMMARY OF THE INVENTION

The present invention is devised to solve the above problems, and therefore, has an object to provide a suitable manufacturing method of epoxides by partially oxidizing unsaturated hydrocarbons in the presence or molecular hydrogen and oxygen by using a catalyst made by depositing fine gold particles on titanium-containing oxides.

The inventors of the present invention has conducted an assiduous study on the conventional manufacturing method of epoxides, and completed the present invention when they discovered that, as to the manufacturing method of epoxides by partially oxidizing unsaturated hydrocarbons in the presence of molecular hydrogen and oxygen by using a catalyst made by depositing fine gold particles on titanium-containing oxides, by partially oxidizing unsaturated hydrocarbons in the presence of a predetermined quantity of a co-existing compound of a particular kind (in other words, in a co-existence of a predetermined quantity of a co-existing compound of a particular kind in a reaction system) , (1) the catalyst shows high activity and selectivity; (2) consumption of hydrogen can be reduced compared with the conventional method; and (3) the foregoing excellent catalytic performance can be maintained over a considerable period in a stable manner (in other words, the catalytic performance is hardly deteriorated with time).

In other words, in order to solve the above problems, the manufacturing method of epoxides of the present invention is a manufacturing method of epoxides by partially oxidizing unsaturated hydrocarbons in the presence of molecular hydrogen and oxygen by using a catalyst made by depositing fine gold particles on titanium-containing oxides, wherein the partial oxidation is carried out in the presence of alcohols and/or ethers in a volume ratio of 10⁻⁴-50 with respect to 1 molecular hydrogen.

According to the above method, the reaction can be carried out under the conditions that (1) the catalyst shows high activity and selectivity; (2) consumption of hydrogen can be reduced compared with the conventional method; and (3) the foregoing excellent catalytic performance is hardly deteriorated with time and therefore can be maintained over a considerable period in a stable manner. In addition, even if a volume ratio of unsaturated hydrocarbons with respect to molecular hydrogen is smaller than a generally known suitable ratio in manufacturing epoxides, the reaction can be carried out efficiently. In other words, an effect of the present invention is to provide a manufacturing method of epoxides capable of reducing consumption of hydrogen compared with the conventional method, and obtaining epoxides at high yield and selectivity over a considerable period.

### DESCRIPTION OF THE EMBODIMENTS

A manufacturing method of epoxides of the present invention is a manufacturing method of epoxides by partially oxidizing unsaturated hydrocarbons in the presence of molecular hydrogen and oxygen by using a catalyst made by fixing fine gold particles on titanium-containing oxides, wherein the partial oxidation is carried out in the presence of a predetermined quantity of alcohols and/or ethers used as a co-existent compound.

The following will explain in the first place a catalyst (hereinafter, referred to as epoxide manufacturing catalyst or simply as catalyst in some cases) made by fixing fine gold particles on titanium-containing oxides used in the manufacturing method of epoxides of the present invention.

Examples of titanium-containing oxides include at least one kind of compound selected from the group consisting of titanium oxides, titanates, and complex oxides made by chemically bonding titanium to silicon by means of oxygen. One member of a mixture of two or more members selected from these examples can be used effectively as the titanium-containing oxides. Of all the examples of titanium-containing oxides, the crystalline structure, shape, size, etc. of titanium oxides are not especially limited.

Titanium oxides may be used independently, but it can be used in the form of a mixture made by physically mixing titanium oxides with silicon-containing oxides, that is, a physical mixture of titanium oxides and silicon-containing oxides (hereinafter, referred to as mixed oxides). The mixed oxides are preferably porous compounds with a specific surface area of 1 m²/g or greater, or preferably in a range from 50 m²/g to 1200 m²/g. Examples of silicon-containing oxides forming the mixed oxides include: compounds mainly composed of silicon oxides, such as amorphous silica and silicalite having a crystallizing property; complex oxides made of silicon and other kinds of metals, such as amorphous silica-alumina and silica-alumina having a crystallizing property (zeolite); etc.

Examples of titanates include titanates of metals, such as MgTiO₃, CaTiO₃, BaTiO₃, PbTiO₃, and FeTiO₃.

It is preferable that the complex oxides made by chemically bonding titanium to silicon by means of oxygen (hereinafter, referred to simply as complex oxides) are porous compounds with a specific surface area of 1 m²/g or greater, and more preferably in a range from 50 m²/g to 1200 m²/g. The complex oxides of titanium and the foregoing silicon-containing oxides are more preferable than the others, and examples of which include: complex oxides made by depositing titanium oxides on the surface of silicon-containing oxides, complex oxides made by depositing titanium on the surface and the inside of the silicon-containing oxides.

Titanium oxides can be deposited on the surface of silicon-containing oxides by general methods, by which titanium compounds are absorbed or bonded to the surface of silicon-containing oxides by means of impregnation, ion-exchange, chemical vapor deposition, etc., and the titanium compounds are turned into oxides by means or calcination, so that titanium oxides are deposited on the surface of the silicon-containing oxides. However, the depositing method is not limited to the foregoing. In addition, complex oxides having titanium deposited on the surface and inside of silicon-containing oxides can be prepared by producing precipitates of hydroxides (mixed hydroxides) containing titanium and silicon out of a homogeneous solution containing titanium compounds and silicon compounds by means of co-precipitation or sol-gel method, and turning the precipitates into oxides by means of calcination or the like.

In case that titanium-containing oxides contain the mixed oxides or complex oxides, that is, in case that titanium-containing oxides contain silicon, an atomic ratio between titanium to silicon (Ti/Si) is preferably in a range from 0.1/100 to 50/100, and more preferably in a range from 0.5/100 to 20/100. A catalyst prepared by using titanium-containing oxides having titanium smaller that the foregoing ratio is not preferable because the catalytic characters are substantially the same as those of a catalyst made by using silica alone as a carrier, thereby failing to partially oxidize unsaturated hydrocarbons selectively.

Fine gold particles are fixed on titanium-containing oxides. So-called ultra-fine gold particles having a particle size of 10 nm or less are suitable as the fine gold particles. A quantity of gold carried on titanium-containing oxides based on a total weight of titanium-containing oxides is preferably 0.001 wt% or greater, more preferably in a range from 0.005 wt% to 20 wt%, further preferably in a range from 0.01 wt% to 20 wt%, particularly preferably in a range from 0.01 wt% to 10 wt%, and most preferably in a range from 0.05 wt% to 10 wt%. A gold carrying quantity of less than 0.001 wt% is not preferable because the activity of the catalyst is deteriorated. On the other hand, a gold carrying quantity over 20 wt% is not preferably either, because the activity of the catalyst is hardly improved compared with a case of depositing the fine gold particles of the foregoing specified ranges, thereby wasting an excessively increased quantity of gold.

Examples of the method of depositing the fine gold particles on titanium-containing oxides, that is, fixing the former on the latter include a precipitation method disclosed in Japanese Laid-open Patent Application No. 8797/1995 (Japanese Official Gazette, *Tokukaihei No. 7*-*8797*, publishing date: January 13, 1995), and a vapor deposition method disclosed in Japanese Laid-open Patent Application No. 122478/1997 (Japanese Official Gazette, *Tokukaihei No. 9-122478*, publishing date: May 13, 1997), and an impregnation method, etc., for example. It should be appreciated, however, that the depositing method is not limited to the foregoing.

The following will explain, of all the foregoing methods, the procedure of depositing the fine gold particles on titanium-containing oxides by means of precipitation. Initially, an aqueous solution containing a gold compound (described below) is prepared, and the aqueous solution is heated to temperatures ranging from 30 °C to 100 °C, and more preferably to temperatures ranging from 50 °C to 95 °C, after which a pH of the aqueous solution is adjusted to be in a range from 6 to 12, and more preferably in a range from 7 to 10 by adding an alkali aqueous solution with stirring. Subsequently, titanium-containing oxides are placed into the aqueous solution with stirring at the foregoing temperature range collectively at one time or more than once within a few minutes. If a pH of the aqueous solution changes, a pH is adjusted to be in the above-specified range by adding the alkali aqueous solution.

After titanium-containing oxides are placed, the aqueous solution is kept stirred at the foregoing temperature range, whereby solid (compound to which fine gold particles are fixed) is obtained as titanium-containing oxides with gold hydroxides being absorbed thereto (deposited thereon). The solid is taken out by means of filtration, washed (rinsed) with water, and calcined at temperatures ranging from 100 °C to 800 °C, whereby the fine gold particles are deposited on titanium-containing oxides.

An alkali component forming the alkali aqueous solution is not especially limited, and examples of such an alkali component include sodium carbonate, potassium carbonate, sodium hydrocarbonate, sodium hydroxide, potassium hydroxide, cesium hydroxide, ammonia, tetramethyl ammonium hydroxide, etc.

The gold compounds are not especially limited, and example of such gold compounds include water-soluble gold salts, such as hydrogen tetrachloroaurate (III) (H[AuCl₄]). A concentration of the gold compounds in a solution, such as an aqueous solution, is not especially limited, either. It should be noted that the gold compounds may be in the form of hydrates.

The epoxide manufacturing catalyst in which the fine gold particles are fixed on titanium-containing oxides can be obtained by the foregoing methods.

The titanium-containing oxides forming the catalyst may further include at least one kind of element selected from the group consisting of alkali metal, alkali earth metal, lanthanoid, and thallium. Examples of alkali metal include lithium, sodium, potassium, rubidium, cesium, and francium. Examples of alkali earth metal include beryllium, magnesium, calcium, strontium, barium, and radium. Examples of lanthanoid include lanthanum, cerium, samarium, etc. One member or a mixture of two or more members selected from these examples can be used effectively as occasion demands. Of all the examples, sodium, potassium, rubidium, cesium, magnesium, calcium, strontium, and barium are more preferable than the others. If titanium-containing oxides further contains the foregoing element(s), not only can the stability of the epoxide manufacturing catalyst be ensured, but also the useful life can be prolonged further. This is the reason why the present invention can offer an effect that epoxides can be obtained at high yield and selectivity over a considerable period.

The method of making titanium-containing oxides contain these elements is not especially limited. For example, when depositing the fine gold particles on titanium-containing oxides by means of precipitation, a compound containing these elements or an aqueous solution thereof (optionally, the foregoing alkali aqueous solution used in adjusting a pH, if alkali metal or alkali earth metal is included therein) can be charged to an aqueous solution containing metal compounds.

In case that titanium-containing oxides include the foregoing elements, a contained quantity of these elements based on a total weight of titanium-containing oxides is preferably in a range from 0.001 wt% to 20 wt%, and more preferably in a range from 0.005 wt% to 5 wt%, and further preferably in a range from 0.01 wt% to 2 wt%. In case that titanium-containing oxides contain titanates, a contained quantity of these elements can be increased from the above-specified ranges. More specifically, a contained quantity is preferably increased to a range from 0.1 wt% to 50 wt%.

Next, the following will explain the manufacturing method of epoxides. The manufacturing method of the present invention is a method, by which unsaturated hydrocarbons are partially oxidized (epoxidation reaction) by using the foregoing catalyst in the presence of molecular hydrogen, oxygen, and a predetermined quantity of alcohols and/or ethers (in other words, in a co-existence of a predetermined quantity of alcohols and/or ethers with the reaction series).

In the above manufacturing method, unsaturated hydrocarbons used as a raw material can be any compound having an olefin double bond. However, a compound having 2-12 carbon atoms are preferable. Examples of unsaturated hydrocarbons include olefines, such as ethylene, propylene, 1-butene, 2-butene, isobutene, 1-pentene, 2-pentene, 2-methyl-1-butene, 3-methyl-1-butene, cyclopentene, 1-hexene, 2-hexene, 3-hexene, 2-methyl-1-pentene, 3-methyl-1-pentene, 4-methyl-1-pentene, cyclohexene, 1-methyl-1-cyclopentne, and 3-methyl-1-cyclopentene. Corresponding epoxides are produced when the double bond of unsaturated hydrocarbons is oxidized. If unsaturated hydrocarbons of the foregoing examples are used as a raw material, the resulting catalyst can have excellent activity and selectivity while consuming less hydrogen during the reaction. Moreover, the resulting catalyst can maintain the catalytic performance over a considerable period.

Molecular hydrogen functions as a reducing agent. A quantity of hydrogen employed is not especially limited, and a suitable quantity in terms of a volume ratio to unsaturated hydrocarbons (hydrogen/unsaturated hydrocarbons) is in a range from 1/10 to 100/1. Because the larger the ratio of hydrogen, the faster the reaction rate, it is preferably that the volume ratio is in closer proximity to 100/1.

A quantity of oxygen employed is not especially limited, either, and a suitable quantity in terms of a volume ratio to unsaturated hydrocarbons (oxygen/unsaturated hydrocarbons) is in a range from 1/10 to 10/1. Using oxygen in a quantity smaller than the above range is not preferable, because the yield of epoxides is reduced. On the other hand, using oxygen in a quantity over the above range is not preferable either, because the yield of epoxides is hardly improved compared with a case using oxygen within the above range while the selectivity is reduced.

A quantity of epoxide manufacturing catalyst employed is not especially limited, and can be set according to a quantity of loadings of fine gold particles, the kind of unsaturated hydrocarbons, reaction conditions, etc. However, a suitable quantity is such that a space velocity (SV) of unsaturated hydrocarbons during the reaction is in a range from 100 hr⁻¹·ml/g·cat. to 30,000 hr⁻¹·ml/g·cat. (a space velocity per 1 g of catalyst).

Examples of alcohols include, but not limited to: lower alcohols having 6 or less carbon atoms, such as methyl alcohol, ethyl alcohol, n-propyl alcohol, isopropyl alcohol, n-butyl alcohol, sec-butyl alcohol, and isobutyl alcohol.

Examples of ethers include: straight-chain ethers, such as dimethyl ether, diethyl ether, and methyl ethyl ether; cyclic ethers, such as dioxiane, tetrahydrofuran, and trioxiane; etc.

One member or a mixture of two or more members selected from these examples can be effectively used as alcohols or ethers. Also, a mixture of alcohols and ethers can be used as well.

A quantity of alcohols and/or ethers (hereinafter, referred to as co-existing compound in some cases) which co-exist with the reaction series in terms of a volume ratio to molecular hydrogen (co-existing compound/molecular hydrogen) is in a range from 1 × 10⁻⁴ to 50, more preferably in a range from 5 × 10⁻⁴ to 40, and further preferably in a range from 1 × 10⁻³ to 20. If the volume ratio is smaller than 1 × 10⁻⁴, no effect is offered by co-existing the co-existing compound with the reaction series, and a quantity of produced epoxides (activity of the catalyst) is reduced sharply. On the other hand, if the volume ratio exceeds 50, the co-existing compound causes active site poisoning and combustion or partial oxidation of the co-existing compound occurs predominately, thereby reducing a quantity of produced epoxides sharply as well.

A method and timing of co-existing the co-existing compound with the reaction series are not especially limited. For example, ① the catalyst is pre-treated by the co-existing compound and then the epoxidation reaction is carried out over the pre-treated catalyst, ② the co-existing compound in a vapor state is supplied to the catalyst together with a raw material gas including an unsaturated hydrocarbons gas, hydrogen and oxygen, and ③ carrying out the methods ① and ② together, etc.

To pre-treat the catalyst by the co-existing compound means, for example, (a) the co-existing compound is impregnated into the catalyst before the epoxidation reaction, and an excessive co-existing compound is removed by heating or the like, (b) the co-existing compound in a vapor state is supplied immediately before the epoxidation reaction together with an inert gas or an oxygen-containing inert gas, etc. By using the catalyst pre-treated in the above manner, the epoxidation reaction of unsaturated hydrocarbons can be carried out suitably.

As has been discussed, by co-existing alcohols and/or ethers with the reaction series in the above-specified volume ratio to molecular hydrogen, not only can epoxides be obtained at high yield and selectivity, but also deterioration of the catalytic performance with time can be prevented, thereby maintaining excellent catalytic performance over a considerably period in a stable manner. In other words, the above arrangement can offer an effect that epoxides can be manufactured at an industrial level over a considerable period.

The reason why such an effect is offered is not clear. However, the followings are presumed as the reasons:
(1) alcohols and/or ethers suppress a polymerization of produced epoxides or an isomerization to aldehyde by reacting with or blocking acidic sites on the catalyst (for example, acidic hydroxyl groups on the surface of the catalyst (silanol hydroxyl group when the catalyst contains silicon)) ; and
(2) because a polymerization of epoxides is suppressed, active site poisoning caused by compound having a high boiling point, such as epoxides polymerization (epoxy polymers), is suppressed as well; etc.

Also, although the reason why is not clear either, by co-existing alcohols and/or ethers with the reaction series in the above specified volume ratio to molecular hydrogen, consumption of hydrogen can be reduced compared with the conventional method. In addition, the reaction can be carried out efficiently even if a volume ratio of molecular hydrogen to unsaturated hydrocarbons is smaller than a generally known suitable volume ratio in manufacturing epoxides.

It is preferable to conduct the manufacturing method of the present invention, that is, carry out a partial oxidation of unsaturated hydrocarbons (epoxidation) , in a vapor phase. To be more specific, a suitable method is such that a reactor is filled with the epoxide manufacturing catalyst, and a raw material gas (optionally together with the co-existing compound) is flown through the reactor, whereby a gas containing the target object, that is, epoxides, can be produced. The raw material gas may be diluted as necessary with an inert gas, such as nitrogen, helium, argon, and carbon dioxide. A quantity of used inert gas is not especially limited. The reaction method is not especially limited, either, but a continuous method is preferable because the above reaction is a so-called gas phase heterogeneous catalyst reaction.

A reaction temperature is not especially limited when the epoxidation is carried out in a vapor phase, and can be set arbitrary depending on the kind of unsaturated hydrocarbons and a combination with the epoxide manufacturing catalyst, etc. However, a temperature at which unsaturated hydrocarbons and epoxides can remain in a vapor state, for example, a range from 0°C to 350°C is suitable, and a range from 20°C to 280°C is most preferable. A too low reaction temperature is not preferable, because yield of epoxides is reduced. On the other hand, a too high reaction temperature is not preferable either, because unsaturated hydrocarbons and epoxides are completely oxidized, and unwanted carbon dioxide and water are generated. Thus, not only selectivity of epoxides is reduced, but also a quantity of consumed hydrogen is increased.

A reaction pressure is not especially limited and can be set arbitrary depending on the reaction conditions, such as the reaction temperature. However, a pressure at which unsaturated hydrocarbons and epoxides can remain in a vapor state, for example, a range from 0.01 MPa to 10 MPa, is preferable. A too low reaction pressure is not preferable, because the yield of epoxides is reduced. On the other hand, a too high reaction pressure is not practical (applicable for industrial use), because although the yield of epoxides is increased, an extra facility, such as a compressor, is required. A reaction time is not especially limited and can be set depending on the reaction conditions, such as the reaction temperatures and reaction pressures.

The reaction of partially oxidizing unsaturated hydrocarbons can be carried out in a liquid phase, and any of reaction types including batch, semi-batch, continuous flow types can be adopted. Preferable reaction temperature and reaction pressure in the liquid phase reaction are a temperature and a pressure at which unsaturated hydrocarbons and epoxides can remain in a vapor state, for example, at 150°C or below and in a range from 0.05 MPa to 10 MPa, respectively. Alternatively, the reaction may be carried out in the liquid phase by using a solvent inactive to the reaction. Methods of using the solvent are not especially limited, and preferred is a method, by which the raw material gas is bubbled in a suspension liquid prepared by suspending the epoxide manufacturing catalyst in a solvent. The solvent is not especially limited, and examples of such a solvent include aromatic hydrocarbons, such as benzene, and a hydrocarbon halide, such as methylene chloride. A used quantity of the solvent is not especially limited, either.

Alcohols and/or ethers added to the reaction series as the co-existing compound are partially and selectively oxidized by the catalyst, and turned into aldehydes and ketones. However, a major portion of the same is not subjected to the oxidation, and can be separated from the reaction product (product gas) when the reaction ends, and collected to be recycled.

If a catalyst used for a considerable time is calcined in the air, the original catalytic performance is restored, and therefore, the catalyst can be used repetitively. Here, it is preferable to calcine the catalyst under the conditions that alcohols and/or ethers described as the co-existing compound co-exist with air.

The following description will describe the present invention in detail by way of Examples and Comparative Examples without any intention to limit the scope of the present invention.

### (Example 1)

After 60 g of silicon oxide commercially known as SILICA Q-10 of Fuji Silysia Chemical Ltd. (specific surface area: 326 m²/g, 10 mesh to 20 mesh, particle size: 840 µm to 1,700 µm) as silicon-containing oxide was dipped into 500 ml of methyl alcohol solution containing 1.96 g of titanium oxide (II) acetyl acetonate, methyl alcohol was removed by using an evaporator. The resulting solid was dried at 120 °C for 12 hours, and subsequently calcined in the air at 600 °C for three hours, whereby silicon oxide having titanium oxide supported thereon was obtained as titanium-containing oxide (complex oxide).

Then, 0.344 g of hydrogen tetrachloroaurate (III) as the gold compound was dissolved into water, and pH of the aqueous solution was adjusted to 8.8 by adding sodium hydroxide aqueous solution (alkali-metal-containing aqueous solution), whereby 500 ml of an aqueous solution of hydrogen tetrachloroaurate (III) was prepared. Subsequently, 40 g of silicon oxide having titanium oxide supported thereon was charged into the aqueous solution, and aqueous solution was stirred for one hour under the temperature condition of 70 °C, whereby silicon oxide having titanium oxide supported thereon was suspended and gold precipitants (fine gold particles) were fixed onto the surface thereof.

Then, the suspension liquid was filtered and the residue, that is, the solid (compound with fine gold hydroxide particles being fixed thereon) was washed with water and dried. Subsequently, the solid was calcined in the air at 400 °C for three hours, whereby a gold catalyst, which is a catalyst comprising titania-silica having gold deposited thereon (hereinafter, referred to simply as catalyst), was obtained.

Then, a stainless reaction cell with an inside diameter of 8 mm was filled with 1 g of the catalyst. Subsequently, a partial oxidation reaction of propylene (unsaturated hydrocarbons) was carried out while keeping the temperature of a catalyst layer (catalyst temperature during heating of the catalyst layer, hereinafter, referred to as reaction temperature) at 200 °C, and the performance of the catalyst was analyzed. More precisely, a raw material gas was prepared by mixing propylene, hydrogen, oxygen, methyl alcohol (co-existing compound) , and argon in a volume ratio (propylene/hydrogen/oxygen/methyl alcohol/argon) of 20/20/20/1/39. Then, the raw material gas was supplied to the catalyst layer in the reaction cell kept at 200 °C at a flowing rate of 5,000 hr⁻¹·ml/g·cat. (converted value at normal temperature under normal pressure) , whereby an epoxidation reaction of propylene was carried out.

The product gas from an outlet of the reaction cell was sampled after one hour and three hours since the reaction had started, and the composition of the sample was analyzed by means of gas chromatography.

Then, the reaction temperature was raised to 210 °C, and the composition of the product gas was analyzed after another one hour and three hours (that is, four hours and six hours since the reaction had started, respectively). In addition, the reaction temperature was raised to 220 °C, and the composition of the product gas was analyzed after another one hour and three hours (that is, seven hours and nine hours since the reaction had started, respectively). From the analyses of the composition of the product gas, one-pass yield of propyleneoxide (epoxides) and a molar ratio (hydrogen/propylene oxide) of consumed hydrogen and produced propyleneoxide were obtained. It can be judged that consumption of hydrogen is reduced as the molar ratio is in closer proximity to 1. The analyses of the product gas are shown in Table 1 together with major reaction conditions. In the reaction conditions, a volume ratio of the co-existing compound means a volume of the co-existing compound when 1 is given as the volume of (molecular) hydrogen in the raw material gas.

### (Example 2)

Prior to the epoxidation reaction of propylene, the pre-treatment was carried out to the catalyst obtained in Example 1 by using methyl alcohol as the co-existing compound. The following will describe the details of the pre-treatment.

Initially, methyl alcohol in a vapor state was mixed (present together) with a helium gas as an inert gas, whereby a treating gas was prepared. A partial pressure of methyl alcohol in the treating gas was 100 mmHg. Then, a stainless reaction cell with an inside diameter of 8 mm was filled with 1g of the same catalyst as Example 1, and the treating gas was supplied to the catalyst layer kept at 200 °C at a flow rate of 83 ml/m for 20 minutes. Then, a helium gas was supplied to the catalyst layer for five minutes to remove methyl alcohol left in the reaction cell. Subsequently, the raw material gas used in Example 1 was supplied to the pre-treated catalyst, and the epoxidation reaction was carried out and the performance of the catalyst was analyzed in the same manner as Example 1. The analyses of the product gas are shown in Table 1 together with major reaction conditions.

### (Comparative Example 1)

The catalyst was prepared in the same manner as Example 1. Then, the epoxidation reaction of propylene was carried out and the performance of the catalyst was analyzed in the same manner as Example 1 except that propylene, hydrogen, oxygen, and argon forming the raw material gas were mixed in a volume ratio (propylene/hydrogen/oxygen/argon) of 20/20/20/40, and that a vapor state of methyl alcohol (co-existing compound) was further mixed in such a manner that the concentration of methyl alcohol in the raw material gas was 10 ppm. The analyses of the product gas are shown in Table 1 together with major reaction conditions.

As is apparent from Table 1, by co-existing methyl alcohol (alcohols) as the co-existing compound with the reaction series in a volume ratio of 1 × 10⁻⁴-50 to 1 with respect to molecular hydrogen, the propylene oxide producing activity (one-pass yield) remains stable over a reaction time and a ratio of consumption of hydrogen (molar ratio) is reduced.

### (Examples 3 - 6)

The catalyst was prepared in the same manner as Example 1 in each example. Then, the epoxidation reaction of propylene was carried out and the performance of the catalyst was analyzed in the same manner as Example 1 except that a mixing ratio of the raw material gas was changed as follows.

A volume ratio (propylene/hydrogen/oxygen/methyl alcohol/argon) was 20/20/20/0.01/40 in Example 3, 20/20/20/0.1/39.9 in Example 4, 20/20/20/10/30 in Example 5, and 20/20/20/20/20 in Example 6.

The analyses of the product gases are shown in Tables 2 and 3 together with major reaction conditions.

### (Example 7)

The catalyst was prepared in the same manner as Example 1. Then, the epoxidation reaction of propylene was carried out and the performance of the catalyst was analyzed in the same manner as Example 1 except that a mixing ratio among propylene, hydrogen, oxygen, methyl alcohol (co-existing compound) , and argon in the raw material gas was changed to a volume ratio of 1/1/1/20/77, and the reaction temperature was raised step by step to 120 °C (after 1-3 hours since the reaction had started), 130 °C (after 3-5 hours since the reaction had started), and 140 °C (after 5-7 hours since the reaction had started). The analyses of the product gas are shown in Table 3 together with major reaction conditions.

### (Comparative Example 2)

The catalyst was prepared in the same manner as Example 7. Then, the epoxidation reaction of propylene was carried out and the performance of the catalyst was analyzed in the same manner as Example 7 except that a mixing ratio among propylene, hydrogen, oxygen, methyl alcohol (co-existing compound), and argon forming the raw material gas was changed to a volume ratio of 1/1/1/60/37. The analyses of the product gas are shown in Table 3 together with major reaction conditions.

Table 3 reveals that the reactivity was deteriorated when a volume ratio of methyl alcohol as the co-existing compound to molecular hydrogen is outside of the above specific range of 1 × 10⁻⁴-50.

### (Example 8)

After 60 g of silicon oxide commercially known as SILICA Q-10 of Fuji Silysia Chemical Ltd. (specific surface area: 326 m²/g, 10 mesh to 20 mesh, particle size: 840 µm to 1,700 µm) as silicon-containing oxide was dipped into 500 ml of methyl alcohol solution containing 0.98 g of titanium oxide (II) acetyl acetonate, methyl alcohol was removed by using an evaporator. The resulting solid was dried at 120 °C for 12 hours, and subsequently calcined in the air at 900 °C for three hours, whereby silicon oxide having titanium oxide supported thereon was obtained as titanium-containing oxide (complex oxide). The titanium-containing oxide was carrying 0.5 wt% of titanium oxide.

Then, 0.344 g of hydrogen tetrachloroaurate (III) as the gold compound and 0.02 g of cerium chloride (lanthanoid-containing compound) were dissolved into water, and a pH of the aqueous solution was adjusted to 8.8 by adding sodium hydroxide aqueous solution (alkali-metal-containing aqueous solution) , whereby 900 ml of an aqueous solution of hydrogen tetrachloroaurate (III) was prepared.

subsequently, 20 g of silicon oxide having titanium oxide supported thereon was charged into the aqueous solution, and aqueous solution was stirred for one hour under the temperature condition of 70 °C, whereby titanium oxide carrying silicon oxide was suspended and gold precipitants (fine gold particles) were fixed onto the surface thereof.

Then, the suspension liquid was filtered and the residue, that is, the solid (compound with fine gold particles being fixed thereon) was washed with water and dried. Subsequently, the solid was calcined in the air at 400 °C for three hours, whereby a gold catalyst, which is a catalyst comprising titania-silica having gold deposited thereon (hereinafter, referred to simply as catalyst) was obtained.

Then, a stainless reaction cell with an inside diameter of 8 mm was filled with 1 g of the catalyst, an epoxidation reaction of propylene (unsaturated hydrocarbons) was carried out. The performance of the catalyst was analyzed in the same manner as Example 1 except that a mixing ratio among propylene, hydrogen, oxygen, ethyl alcohol (co-existing compound), and argon in the raw material gas was changed to a volume ratio of 20/20/20/0.6/39.4. The analyses of the product gas are shown in Table 4 together with major reaction conditions.

### (Comparative Example 3)

The catalyst was prepared in the same manner as Example 8. Then, the epoxidation reaction of propylene was carried out and the performance of the catalyst was analyzed in the same manner as Example 8 except that propylene, hydrogen, oxygen, and argon forming the raw material gas was mixed in a volume ratio of 20/20/20/40, and that a vapor state of ethyl alcohol (co-existing compound) was further mixed in such a manner that the concentration of methyl alcohol in the raw material gas was 15 ppm. The analyses of the product gas are shown in Table 4 together with major reaction conditions.

### (Example 9)

The catalyst was prepared in the same manner as Example 8 except that cerium chloride was replaced with strontium chloride hexahydrate (alkali-earth-metal-containing compound), and potassium hydroxide aqueous solution was replaced with cesium hydroxide aqueous solution (alkali-metal-containing aqueous solution). Also, the epoxidation reaction of propylene was carried out and the performance of the catalyst was analyzed in the same manner as Example 8 except that ethyl alcohol used as the co-existing compound was replaced with isopropyl alcohol (IPA), and a mixing ratio among propylene, hydrogen, oxygen, IPA, and argon in the raw material gas was changed to a volume ratio of 20/20/20/2/38. The analyses of the product gas are shown in Table 4 together with major reaction conditions.

### (Comparative Example 4)

The catalyst was prepared in the same manner as Example 9. Then, the epoxidation reaction of propylene was carried out and the performance of the catalyst was analyzed in the same manner as Example 9 except that a mixing ratio among propylene, hydrogen, oxygen, and argon forming the raw material gas was changed to a volume ratio of 20/20/20/40, and that a vapor state of IPA (co-existing compound) was further mixed in such a manner that the concentration of IPA in the raw material gas is was 15 ppm. The analyses of the product gas are shown in Table 5 together with major reaction conditions.

### (Example 10)

The catalyst was prepared in the same manner as Example 1. Then, the epoxidation reaction of propylene was carried out and the performance of the catalyst was analyzed in the same manner as Example 1 except that methyl alcohol used as the co-existing compound was replaced with tetrahydrofuran (THF), and a mixing ratio among propylene, hydrogen, oxygen, THF, and argon in the raw material gas was changed to a volume ratio of 20/20/20/0.5/39.5. The analyses of the product gas are shown in Table 5 together with major reaction conditions.

### (Example 11)

The catalyst was prepared in the same manner as Example 1. Then, the epoxidation reaction of propylene was carried out and the performance of the catalyst was analyzed in the same manner as Example 1 except that a mixing ratio among propylene, hydrogen, oxygen, methyl alcohol, and argon in the raw material gas was changed to a volume ratio of 20/8/20/1/51. The analyses of the product gas are shown in Table 5 together with major reaction conditions.

As can be understood from Table 5, by co-existing methyl alcohol (alcohols) with the reaction series, the reaction can be carried out efficiently even if a volume ratio among molecular hydrogen to unsaturated hydrocarbons (propylene, herein) is lower than a generally known suitable ratio in producing epoxides (See Example 11, in particular).

### (Comparative Example 5)

The catalyst was prepared in the same manner as Example 11. Then, the epoxidation reaction of propylene was carried out and the performance of the catalyst was analyzed in the same manner as Example 11 except that propylene, hydrogen, oxygen, and argon forming the raw material gas were mixed in a volume ratio of 20/8/20/51, and further a vapor state of methyl alcohol (co-existing compound) was mixed in such a manner that a concentration of methyl alcohol in the raw material gas was 7 ppm. The analyses of the product gas are shown in Table 6 together with major reaction conditions.

### (Example 12)

After 100 g of silicon oxide commercially known as SILICA Q-10 of Fuji Silysia Chemical Ltd. (specific surface area: 326 m²/g, 10 mesh to 20 mesh, particle size: 840 µm to 1,700 µm) as silicon-containing oxide was dipped into 150 ml of methyl alcohol solution containing 3.28 g of titanium oxide (II) acetyl acetonate, methyl alcohol was removed by using an evaporator. The resulting solid was dried at 120 °C for 12 hours, and subsequently calcined in the air at 600 °C for three hours, whereby silicon oxide having titanium oxide supported thereon was obtained as titanium-containing oxide (complex oxide). The titanium-containing oxide was carrying 1 wt% of titanium oxide.

Then, 0.69 g of hydrogen tetrachloroaurate (III) as the gold compound was dissolved into water, and a pH of the aqueous solution was adjusted to 8.8 by adding sodium hydroxide aqueous solution (alkali-metal-containing aqueous solution), whereby 2000 ml of an aqueous solution of hydrogen tetrachloroaurate (III) was prepared.

Subsequently, 40 g of silicon oxide having titanium oxide supported thereon was charged into the aqueous solution, and aqueous solution was stirred for one hour under the temperature condition of 70 °C, whereby titanium oxide carrying silicon oxide was suspended and gold precipitants (fine gold particles) were fixed onto the surface thereof.

Then, the suspension liquid was filtered and the residue, that is, the solid (compound with fine gold particles being fixed thereon) was washed with water and dried. Subsequently, the solid was calcined in the air at 400 °C for three hours, whereby a gold catalyst, which is a catalyst comprising titania-silica having gold deposited thereon (hereinafter, referred to simply as catalyst) was obtained. The quantities of gold and sodium (metal) carried by the catalyst were analyzed by means of x-ray fluorescence analysis. Then, the catalyst was carrying 0.153 wt% of gold and 0.115 wt% of sodium.

Then, a stainless reaction cell with an inside diameter of 8 mm was filled with 1 g of the catalyst. Subsequently, a partial oxidation reaction of trans-2-butene was carried out while keeping the temperature (reaction temperature) of a catalyst layer at 180 °C, and the performance of the catalyst was analyzed. More precisely, a raw material gas was prepared by mixing trans-2-butene, hydrogen, oxygen, methyl alcohol (co-existing compound), and argon in a volume ratio or 5/20/20/2/53. Then, the raw material gas was supplied to the catalyst layer in the reaction cell kept at 180 °C at a flow rate of 4,000 hr⁻¹·ml/g·cat. (converted value at normal temperature under normal pressure) , whereby an epoxidation reaction of trans-2-butene was carried out.

The product gas from an outlet of the reaction cell was sampled after one hour and three hours since the reaction had started, and the composition of the sample was analyzed by means of gas chromatography.

Then, the reaction temperature was raised to 190 °C, and the composition of the product gas was analyzed after another one hour and three hours (that is, four hours and six hours since the reaction had started, respectively). In addition, the reaction temperature was raised to 200 °C, and the composition of the product gas was analyzed after another one hour and three hours (that is, seven hours and nine hours since the reaction had started, respectively). From the analyses of the composition of the product gas, one-pass yield of 2,3-epoxybutane (epoxides) and a molar ratio (hydrogen/2,3-epoxybutane) of consumed hydrogen and produced 2,3-epoxybutane were determined. It can be judged that consumption of hydrogen is reduced as the molar ratio is in closer proximity to 1. The analyses of the product gas are shown in Table 6 together with major reaction conditions.

Subsequently, the temperature of the catalyst used for consecutive nine hours was kept at 300 °C, and subjected to heat treatment for one hour by keep supplying a mixed gas of air and methyl alcohol (volume ratio (air/methyl alcohol): 90/2) to the catalyst at a flow rate of 92 ml/min so as to restore catalyst.

The restored catalyst was used in the epoxidation reaction of 2,3-epoxybutane again, and the epoxidation reaction was carried out and the catalyst performance was analyzed in the same manner as above. The product gas was sampled after one hour, three hours, four hours, and six hours since the reaction had started, and the composition of the sample was analyzed. The one-pass yield of 2,3-epoxybutane (epoxides) was 3.2%, 3.1%, 4.6%, and 4,2%, respectively, thereby revealing that the catalyst performance was completely restored (to original).

### (Comparative Example 6)

The catalyst was prepared in the same manner as Example 12. Then, the epoxidation reaction of trans-2-butene was carried out and the performance of the catalyst was analyzed in the same manner as Example 12 except that a mixing ratio among trans-2-butene, hydrogen, oxygen, and argon forming the raw material gas was changed to a volume ratio of 5/20/20/55, and that a vapor state of methyl alcohol (co-existing compound) was further mixed in such a manner that the concentration of methyl alcohol in the raw material gas was 15 ppm. The analyses of the product gas are shown in Table 6 together with major reaction conditions.

### (Example 13)

The catalyst was prepared in the same manner as Example 12. Then, the epoxidation reaction of cis-2-butene (unsaturated hydrocarbons) was carried out and the performance of the catalyst was analyzed in the same manner as Example 12 except that a mixing ratio among cis-2-butene, hydrogen, oxygen, ethyl alcohol (co-existing compound) and argon forming the raw material gas was changed to a volume ratio of 5/20/20/0.5/54.5. The analyses of the product gas are shown in Table 7 together with major reaction conditions.

### (Comparative Example 7)

The catalyst was prepared in the same manner as Example 13. Then, the epoxidation reaction of cis-2-butene was carried out and the performance of the catalyst was analyzed in the same manner as Example 13 except that a mixing ratio among cis-2-butene, hydrogen, oxygen, and argon forming the raw material gas was changed to a volume ratio of 5/20/20/54.5, and that a vapor state of ethyl alcohol (co-existing compound) was further mixed in such a manner that the concentration of ethyl alcohol in the raw material gas was 15 ppm. The analyses of the product gas are shown in Table 7 together with major reaction conditions.

### (Example 14)

The catalyst was prepared in the same manner as Example 1 except that a quantity of hydrogen tetrachloroaurate (III) as the gold compound was increased to 0.688 g and sodium hydroxide aqueous solution was replaced with potassium hydroxide aqueous solution (alkali-metal-containing aqueous solution).

Then, a stainless reaction cell with an inside diameter of 8 mm was filled with 1 g of the catalyst. Subsequently, a partial oxidation reaction of 1-hexene (unsaturated hydrocarbons) was carried out while keeping the temperature (reaction temperature) of a catalyst layer at 170 °C, and the performance of the catalyst was analyzed. More precisely, a raw material gas was prepared by mixing 1-hexene, hydrogen, oxygen, methyl alcohol (co-existing compound) , and argon in a volume ratio of 7.6/18.9/18.9/2.2/52.4. Then, the raw material gas was supplied to the catalyst layer in the reaction cell kept at 170 °C at a flow rate of 3,300 hr⁻¹·ml/g·cat. (converted value at normal temperature under normal pressure), whereby an epoxidation reaction of 1-hexene was carried out.

The product gas from an outlet of the reaction cell was sampled after one hour and three hours since the reaction had started, and the composition of the sample was analyzed by means of gas chromatography.

Then, the reaction temperature was raised to 180 °C, and the composition of the product gas was analyzed after another one hour and three hours (that is, four hours and six hours since the reaction had started, respectively). In addition, the reaction temperature was raised to 190 °C, and the composition of the product gas was analyzed after another one hour and three hours (that is, seven hours and nine hours since the reaction had started, respectively). From the analyses of the composition of the product gas, one-pass yield of 1,2-epoxyhexane (epoxides) and a molar ratio between consumed hydrogen and produced 1,2-epoxyhexane (hydrogen/1,2-epoxyhexane) were obtained. It can be judged that consumption of hydrogen is reduced as the molar ratio is in closer proximity to 1. The analyses of the product gas are shown in Table 7 together with major reaction conditions.

### (Example 15)

The catalyst was prepared in the same manner as Example 14. Then, the epoxidation reaction of 1-hexene was carried out and the performance of the catalyst was analyzed in the same manner as Example 14 except that methyl alcohol used as the co-existing compound was replaced with IPA. The analyses of the product gas are shown in Table 8 together with major reaction conditions.

### (Comparative Example 8)

The catalyst was prepared in the same manner as Example 14. Then, the epoxidation reaction of 1-hexene was carried out and the performance of the catalyst was analyzed in the same manner as Example 14 except that a mixing ratio among 1-hexene, hydrogen, oxygen, and argon forming the raw material gas was changed to a volume ratio of 7.6/18.9/18.9/54.6, and that a vapor state of methyl alcohol (co-existing compound) was further mixed in such a manner that the concentration of methyl alcohol in the raw material gas was 15 ppm. The analyses of the product gas are shown in Table 8 together with major reaction conditions.

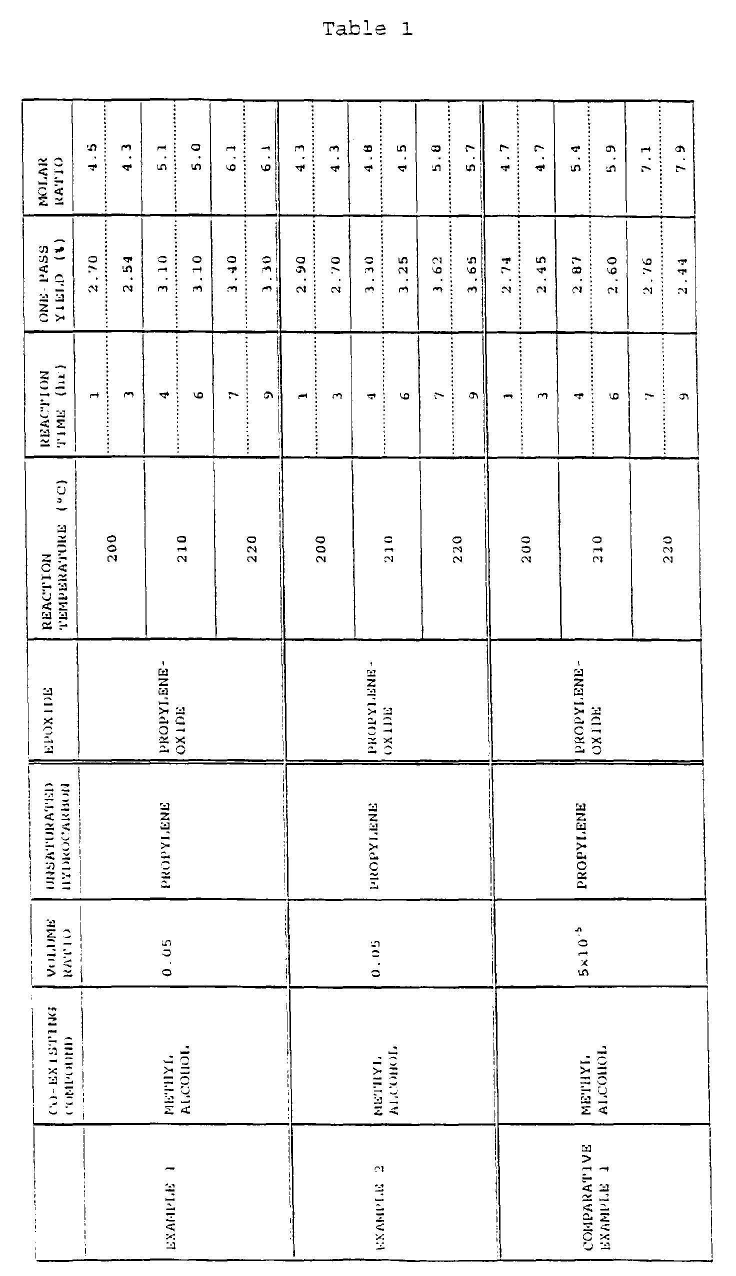

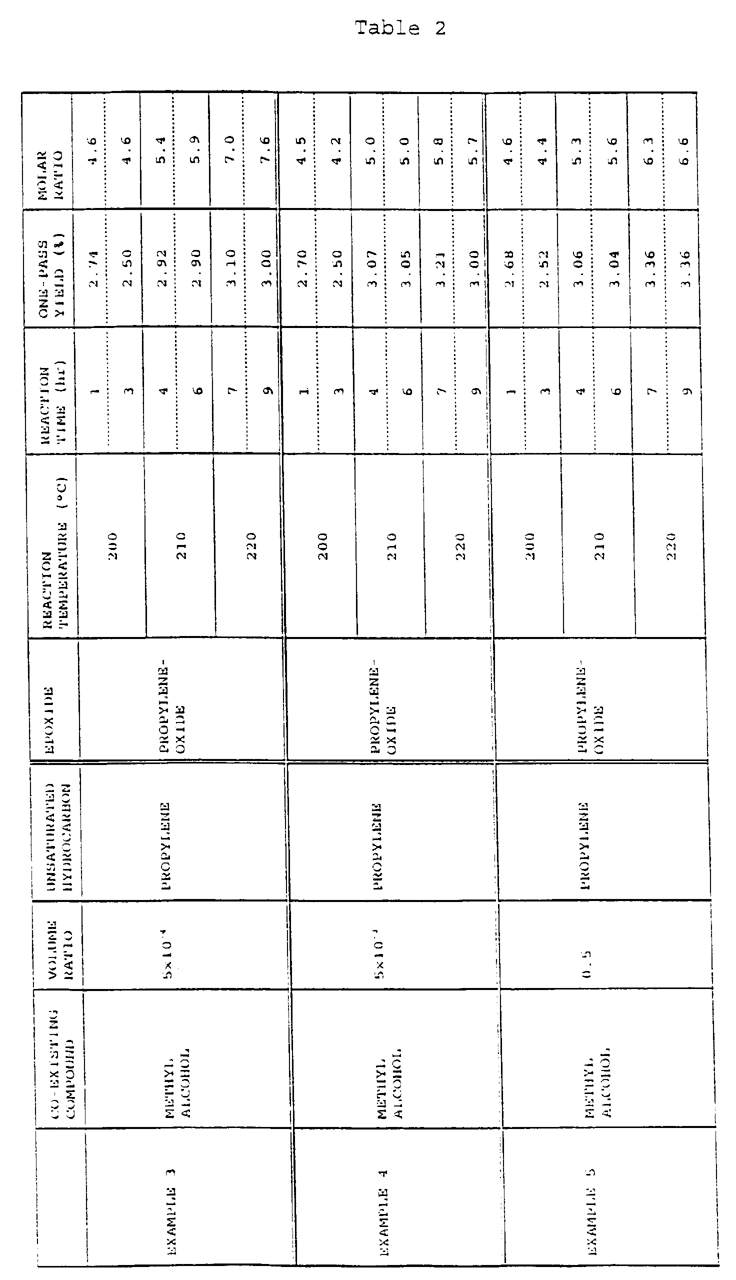

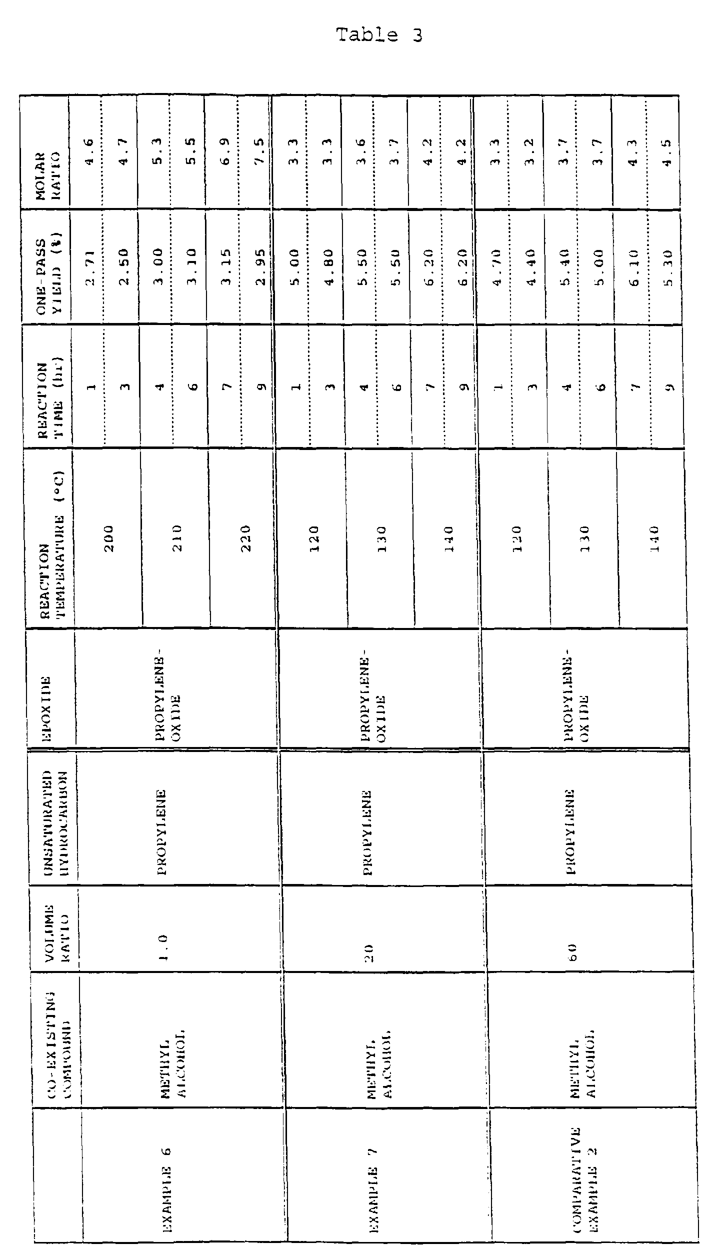

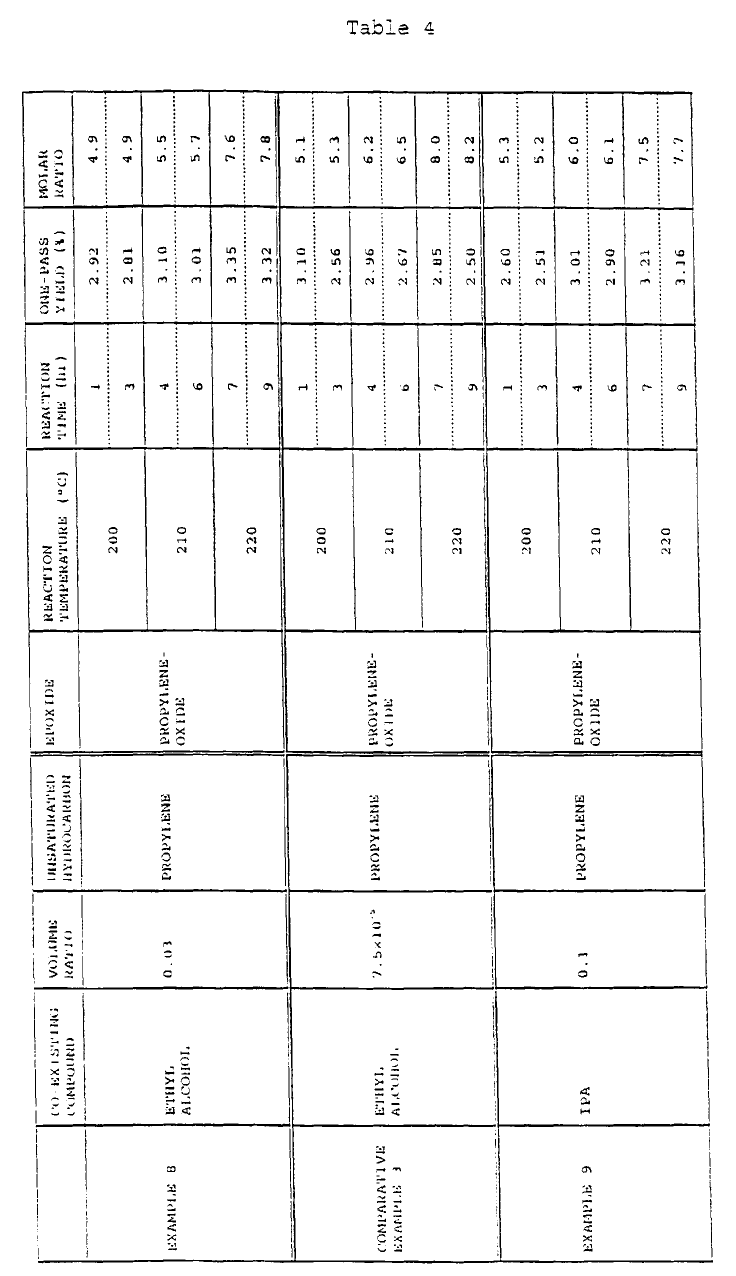

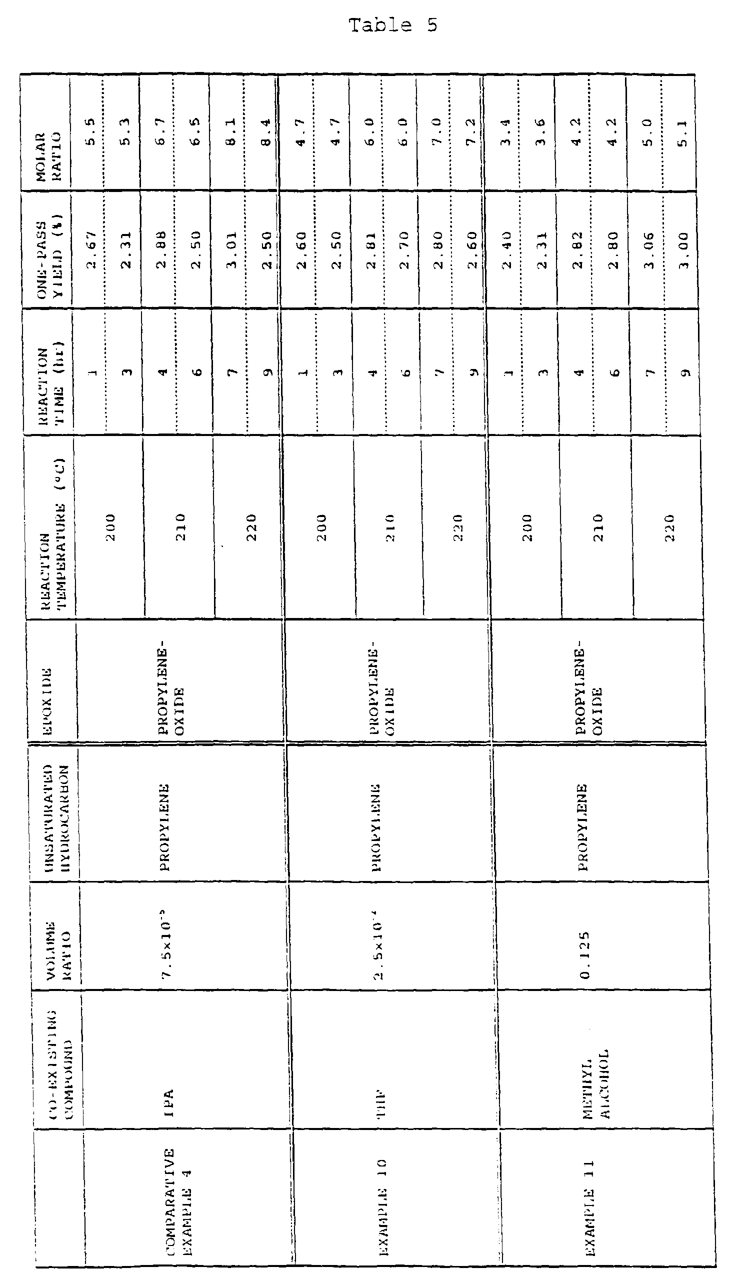

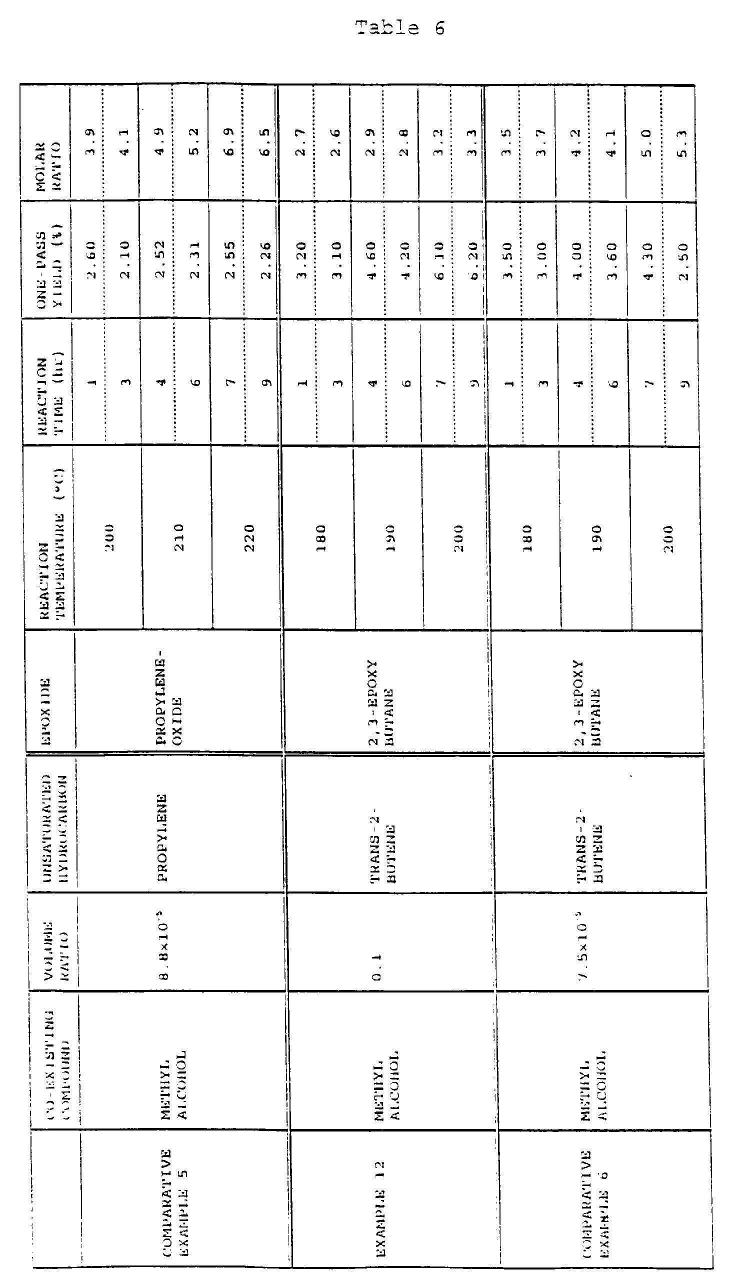

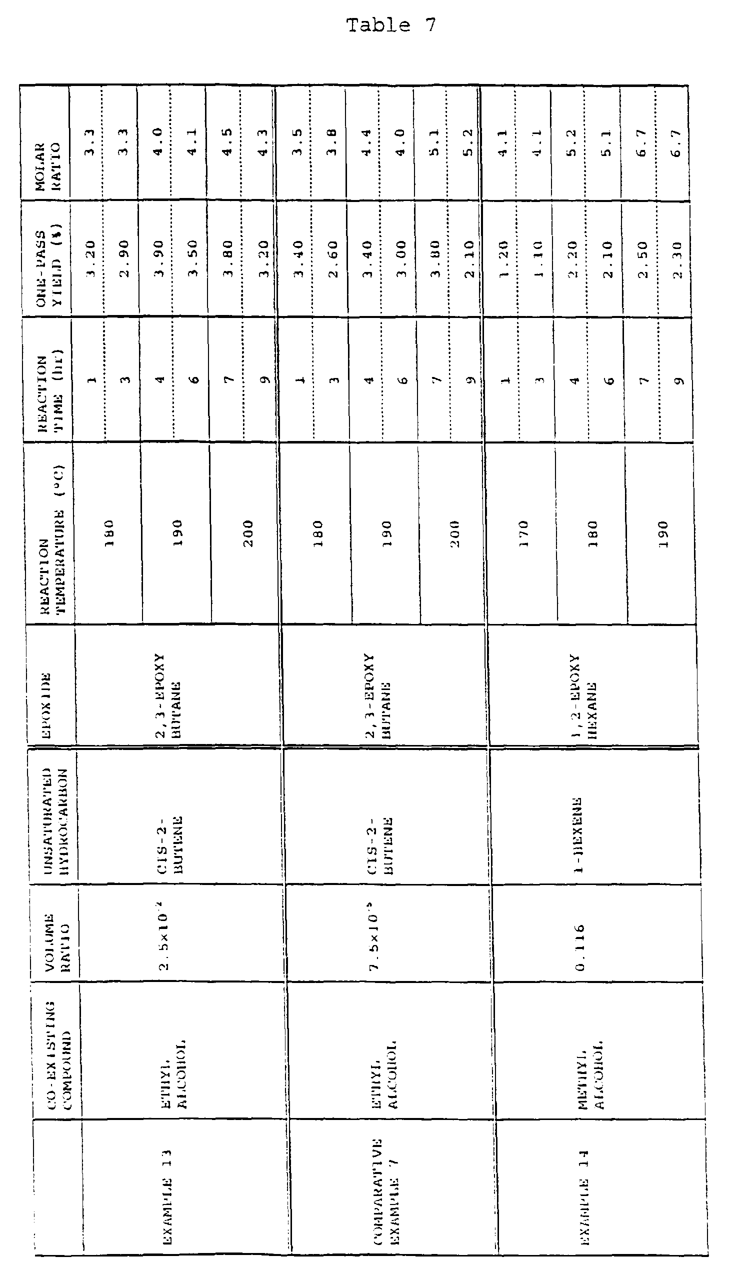

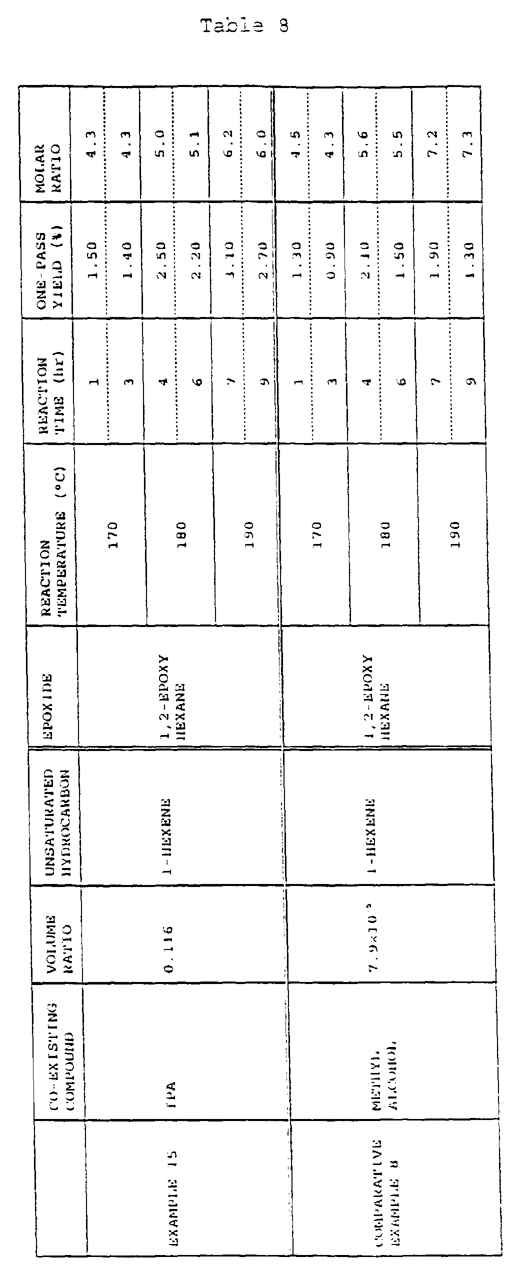

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A manufacturing method of epoxides by carrying out partial oxidation of unsaturated hydrocarbons in the presence of molecular hydrogen and oxygen by using a catalyst made by fixing fine gold particles onto titanium-containing oxides, characterized in that said partial oxidation is carried out in the presence of alcohols and/or ethers in a volume ratio of 1 × 10⁻⁴-50 with respect to 1 molecular hydrocarbons.

2. The manufacturing method of Claim 1, wherein said alcohols are lower alcohols having 6 or less carbon atoms.

3. The manufacturing method of Claim 1 or 2, wherein said titanium-containing oxides are at least one kind of compound selected from the group consisting of titanium oxide, titanate, complex oxide made by chemically bonding titanium to silicon by means of oxygen.

4. The manufacturing method of any of preceding claims, wherein said catalyst includes gold in a range from 0.005 wt% to 20 wt% based on a total weight of said titanium-containing oxides.

5. The manufacturing method of any of preceding claims, wherein a volume ratio between said oxygen and unsaturated hydrocarbons is in a range from 1/10 to 10/1.

6. The manufacturing method of any of preceding claims, wherein a volume ratio between said molecular hydrogen and unsaturated hydrocarbons is in a range from 1/10 to 100/1.

7. The manufacturing method of any of preceding claims, wherein said titanium-containing oxides further include at least one element selected from the group consisting of alkali metal, alkali earth metal, lanthanoid, and thallium.
